# EUROPÄISCHE PATENTSCHRIFT

(11) **EP 1 878 413 B1**
(45) Veröffentlichungstag und Bekanntmachung des Hinweises auf die Patenterteilung: **08.12.2010**
(21) Anmeldenummer: 07013672.6
(22) Anmeldetag: 12.07.2007
(51) Int. Cl.: A61F 13/56

(54) **Verschlussband insbesondere für einen Hygieneartikel, Bandmaterial, Windel und Herstellungsverfahren**
Sealing tape in particular for a sanitary article, bandaging material, nappies and manufacturing method
Bande de fermeture, en particulier pour un article hygiénique, matériau de pansement, couche et procédé de fabrication

(30) Priorität: 12.07.2006 DE 102006032526
(43) Veröffentlichungstag der Anmeldung: 16.01.2008
(73) Patentinhaber: Koester GmbH & Co. KG, 96146 Altendorf (DE)
(72) Erfinder: Degelmann, Walter, Dr., 95326 Kulmbach (DE)
(74) Vertreter: Reuther, Martin

(56) Entgegenhaltungen:
- EP-A- 0 848 938
- WO-A-01/58402
- WO-A-92/01759
- WO-A-03/034966
- WO-A-2004/050970
- GB-A- 2 164 900
- JP-A- 2004 167 025
- US-A- 5 531 731
- US-B1- 6 352 528
- US-B1- 6 524 294

## Beschreibung

Die Erfindung betrifft ein Verschlussband insbesondere für einen Hygieneartikel, ein Bandmaterial, eine Windel und ein Herstellungsverfahren.

Moderne Hygieneartikel, insbesondere Windeln, wie beispielsweise Babywindeln oder Inkontinenzwindeln, weisen Windelverschlussbänder auf. Beim Anlegen einer solchen Windel muss diese über die Hüfte des Trägers gezogen werden. Hierzu wird die Windel in einen geöffneten Anlegezustand gebracht. Sobald die Windel die Position am Körper erreicht hat, in welcher sie getragen werden soll, wird sie geschlossen. Hierzu dienen die Windelverschlussbänder.

In der Regel weist eine Windel zwei Windelverschlussbänder auf, welche jeweils an einem Windelohr permanent befestigt sind. Hierzu dient an einem Windelverschlussband ein Befestigungsbereich. In der Regel liegt hier eine Klebeverbindung vor.

Der Benutzer verschließt die Windel, indem er einen Schließbereich des Windelverschlussbands mit der Oberfläche der Windel lösbar verbindet. Konstruktiv wird dies durch eine Komponente eines Verschlusssystems ermöglicht, welche im Schließbereich angeordnet ist. Meist handelt es sich um einen Hakenbereich eines mechanischen Haken-Schlaufen-Verschlusssystems. An einem Frontaltape der Windel sind Schlaufen vorgesehen, mit welchen die Haken in Eingriff gebracht werden können. Alternativ und kumulativ können auch andere Verbindungsmittel in Betracht kommen, vor allem Klebstoffe.

Die Befestigungsmittel im Befestigungsbereich und die Schließmittel im Schließbereich sind auf derjenigen Seite des Bandes angeordnet, welche bei angelegter Windel zum Träger hin gerichtet sind. Diese Seite des Verschlussbandes wird als Rückseite bezeichnet.

Die körperhafte Struktur eines "Bandes" erhält ein Windelverschlussband durch einen Fasteningträger.

Dieser ist häufig ein Streifen aus einem elastischen, inelastischen oder teilelastischen Werkstoff. Häufig kommen hier Folienstreifen zum Einsatz.

Auf der Vorderseite ist in der Regel ein auf einem Folienstreifen basierender Fasteningträger aus Gründen einer weicheren Haptik und/oder einer gefälligeren Optik in der Regel kaschiert. Je nach der gewünschten Qualität der Oberfläche der Bandvorderseite, also derjenigen Seite, welche an der angelegten Windel sichtbar nach außen gerichtet ist, können hierzu beispielsweise Filamente auf einen stabilen Trägerfilm aufgebracht sein, oder es werden ganze eigenstabile Textilschichten, wie beispielsweise Vliese, auf einen stabilen Trägerfilm auflaminiert.

Es sind auch Fasteningträger mit selbständig textilem Charakter bekannt. Dies sind meist Nonwovens, die entweder eigenstabil oder mit stabilisierenden Mitteln stabil sind. So sind insbesondere eigenstabile Nonwovenlagen bekannt, die zur Rückseite hin eine Befilmung aufweisen können. Diese Befilmung kann die Stabilität des Nonwovens bei geeigneter Gestaltung noch leicht erhöhen. Auch sind Laminate bekannt, die aus einer Verbindung einer eigenstabilen textilen Lage, insbesondere eines Nonwovens, und einer Folie bestehen.

Die Grenze zwischen Fasteningträgern, die im wesentlichen auf einem Folienstreifen basieren, die im wesentlichen auf einer Textillage basieren oder die auf zwei oder mehr Lagen aus Folie, Textillage und/oder Befilmung basieren, ist fließend. Fast ausnahmslos ist jedoch zu beobachten, dass die Vorderseite mit einer textilen Haptik gestaltet wird.

Die Komponente des Verschlusssystems zum öffenbaren Schließen der Windel ist in der Regel, wie bereits erläutert, auf der gegenüberliegenden Rückseite des Fasteningträgers im Schließbereich angeordnet.

Um die Windel für den Benutzer - insbesondere für ein Elternteil im Falle einer Babywindel - besser benutzbar zu machen, werden teilweise Farbmarkierungen am Windelverschlussband vorgesehen. Hierzu ist beispielsweise ein Hakenmaterial aus einem zum übrigen Band kostrastfarbenen Werkstoff im Schließbereich auf die Bandrückseite aufgeklebt. Eine solche farbige Gestaltung der Haken und der Hakenbasis veranlasst den Benutzer intuitiv dazu, das Band in diesem Bereich zum Anlegen anzufassen. Der Benutzer greift somit auch unter widrigen Bedingungen sicher das freie Benutzerende des Windelverschlussbands.

Ein dergestalt farbiges Hakenmaterial kann allerdings nur in großen Mengen kostengünstig hergestellt und somit auch bezogen werden. Liegt eine Charge eines solchen Hakenmaterials einmal vor, wird die Handlungsfreiheit bezüglich der übrigen farbigen Gestaltung der Windelverschlussbänder und/oder der Windeln stark eingeschränkt, weil ansonsten zueinander unpassende Farben an einem Produkt verarbeitet würden. Auch kommt es vor, dass bestimmte Farben eher einem bestimmten Windelanbieter zugeschrieben werden, so dass ein Hakenmaterial mit einer solchen Farbe für einen anderen Windelhersteller nicht brauchbar ist. Auf Seiten des Herstellers des Windelverschlussbandes sind daher große Lagerkapazitäten erforderlich.

Als kostengünstigere Alternative hat sich herausgestellt, gefärbte Folienstreifen auf der Rückseite des Verschlussbands aufzukleben. So ist es beispielsweise bekannt, einen schmalen Folienstreifen am freien Ende eines Windelverschlussbands neben dem Hakenmaterial aufzukleben, also dort, wo der Benutzer das Verschlussband anfassen soll. Eine solche Konstellation, die aufgrund der Verdickung durch die aufgeklebte Folie auch haptisch erkennbar ist, wird als Fingerlift bezeichnet.

Es ist jedoch unmittelbar ersichtlich, dass auch dergestalt gefärbte Folien nur in großen Mengen wirtschaftlich bezogen werden können, so dass wiederum die Flexibilität in der Gestaltung von Windelschlussverschlussbändern und somit von Windeln eingeschränkt ist.

Die Verwendung derartiger Folien innerhalb eines Windelverschlussbandes als Zwischenlage zwischen zwei weiteren Folien, die durchscheinend ausgebildet sein sollen, zeigt die JP 2004-167025 A.

Windelverschlussbänder zeigen unter anderem die DE 28 56 869 A1, die DE 197 32 499 A1, die DE 101 29 180 A1, die DE 101 40 621 A1, die DE 101 40 622 A1, die DE 697 27 584 T2, die EP 0 233 704 A2, die EP 0 233 704 B1, die EP 0 235 014 B1, die EP 0 321 232 B1, die EP 0 418 951 B1, die EP 0 793 953 A2, die EP 0 795 307 A2, die EP 0 800 378 B1, die EP 0 840 585 B1, die EP 0 974 326 B1, die EP 1 000 598 A1, die EP 1 024 774 B1, die EP 1 484 041 A1, die EP 1 484 042 A1, die FR 2 586 558, die FR 2 606 257, die JP 7108-4 C, die US 6,210,389 B1, die US 5,624,429, die US 4,670,012, die US 4,959,265, die US 5,399,219, die US 5,611,790, die US 5,624,429, die US 5,676,652, die US 5,759,317, die US 5,997,522, die US 6,210,389 B1, die US 6,393,673 B1, die US 6,402,730 B1, die US 6,428,525 B1, die US 6,454,751 B1, die US 2002/0095130 A1, die US 2003/0004490 A1, die US 2003/0009144 A1, die US 2003/0023322, die US 2003/0034583 A1, die US 2003/0060794, die US 2004/0127128, die WO 96/31181 A1, die WO 97/23186 A1, die WO 97/32555 A1, die WO 97/36566, die WO 99/06600, die WO 00/27330 A1, die WO 01/67911 A2, die WO 01/68019 A1, die WO 01/74283 A1, die WO 02/49567 A1, die WO 02/49568 A1 und die WO 03/003962 A1.

Der Erfindung liegt die Aufgabe zugrunde, ein Verschlussband zur Verfügung zu stellen, welches eine höhere Flexibilität in der Herstellung erlaubt, ohne jedoch die Komforteigenschaften von Verschlussbändern einzuschränken.

Nach einem ersten Aspekt der Erfindung löst diese Aufgabe ein Verschlussband für einen Hygieneartikel, insbesondere für eine Babywindel oder für eine Inkontinenzwindel, mit einem Fasteningträger mit einer textilen oder textil kaschierten Vorderseite und einer auf einer Rückseite des Fasteningträgers in einem Schließbereich getragenen Komponente eines Verschlusssystems zum lösbaren Verbinden mit einer Oberfläche, wobei der Fasteningträger einen elastischen Träger aufweist, der mit Nonwoven kaschiert ist und sich das Verschlussband durch einen Farbauftrag auf der dem elastischen Träger zugewandten Seite des Nonwoven auszeichnet.

Mit einem Farbauftrag besteht im Herstellungsprozess die Möglichkeit, einheitlich farbige Bestandteile, insbesondere bei deckendem Farbauftrag sogar beliebig farbige mechanische Elemente, zusammenzufügen, wobei erst durch den Farbauftrag das jeweilige mechanische Element ganz oder teilweise seine endgültige Farbe annimmt.

Durch ein Band mit einem rückseitigen Farbauftrag wird daher mit überraschender Simplizität die Farbgestaltung insbesondere der Bandrückseite - also derjenigen Seite, welche der Benutzer bei den Handlungen zum Anlegen der Windel sieht - aus der Verantwortungssphäre des Einkaufs des Bandherstellers genommen und kann auf zeitlich spätere Produktionsstadien verlegt werden. Erfindungsgemäß bleibt der Farbauftrag zu Vorderseite wenigstens durch eine Lage oder Komponente des Verschlussbandes abgedeckt, so dass die Gefahr einer Beeinträchtigung des Farbauftrages, insbesondere an einer Windelmaschine, bei einer geeigneten Ausgestaltung minimiert werden kann. Selbiges gilt auch für eine Beeinträchtigung während des Tragens, da in der Regel die Rückseite des Verschlussbandes am Körper anliegt, während die Vorderseite außen zu finden ist. Insbesondere in letzterer Situation können auch etwaige Abfärbeprozesse minimiert werden.

Der Hersteller eines Windelverschlussbands kann daher große Mengen eines einheitlich eingekauften Ausgangselements verwenden und während der Herstellung zahlreicher Verschlussbänder einfach durch Änderung des Farbauftrags das Endprodukt flexibel verändern.

Diese Verlagerung der Farbgestaltung hin zu späteren Produktionsschritten kann sogar so weit gehen, dass erst der Windelhersteller den Farbauftrag am Verschlussband anbringt, beispielsweise um verschieden farbig gestaltete Windeln herstellen zu können, oder um die Bänder innerhalb einer Windelserie variabel zu gestalten. In diesem Falle verlagern sich sowohl der Vorteil des günstigeren Einkaufs als auch die Vorteile der höheren Produktionsflexibilität vom Hersteller der Verschlussbänder hin zu dem Hersteller der Windel.

Der vom vorgestellten Aspekt der Erfindung eingeschlagene Weg steht in Kontrast zu jeglichen bisherigen Entwicklungen. Der Schritt hin zu gefärbten Folien wurde von der Fachwelt als völlig ausreichende Flexibilisierung des Herstellprozesses angesehen. Ohnehin wurde es teils abgelehnt, die Rückseite nicht in einer einheitlichen Farbgestaltung zu belassen, da bei den meisten Verschlussbändern für Windeln eine farbige Rückseite zur Vorderseite hin mehr oder minder durchscheint. Dies empfand die Fachwelt als kaum vereinbar mit der textilen Oberfläche der Vorderseite, weil diese das durchscheinende Bild in der Farbintensität abschwächt und/oder in den Konturen unscharf macht.

Erst bei Vorliegen der Erfindung wird deutlich, wie groß der Vorteil ist, der in der flexibleren Produktion liegt, wobei der Durchscheineffekt zur Vorderseite hin bei geeigneter Gestaltung nicht störend ist, ja sogar wünschenswert sein kann, da sich überraschender Weise herausgestellt hat, dass Durchscheineffekte zu einer sehr angenehmen Optik auch an der Vorderseite des Verschlussbandes führen.

Gleichzeitig zu dieser Erkenntnis sieht der vorgestellte Aspekt der Erfindung einen rückseitigen "Auftrag" von Farbe vor, wohingegen bislang nur gefärbte Elemente in Form von Haken oder Folien dort angewendet wurden. Es ist aber erheblich einfacher, eine Farbe auf ein Element aufzutragen als ein durchgefärbtes Element herzustellen. Dies ist besonders dann wichtig, wenn - wie hier - gerade nicht die Produktionsmöglichkeiten eines spezialisierten Folienherstellers in Betracht gezogen werden sollen, sondern die Möglichkeiten eines Verschlussbandherstellers oder sogar eines Windelherstellers.

Ein Farbauftrag ist in einem einfachen Fall ein Aufdruck. Hierzu kommen beispielsweise verschiedene Tinten in Betracht. Insgesamt lassen sich zahlreiche bekannte Drucktechniken oder Techniken zum Bestreichen oder Belegen mit Farbe ohne weiteres auf die Kunststoffe an Windelverschlussbändern anwenden.

Zunächst sei erläutert, wo ein rückseitiger Farbauftrag besonders vorteilhaft eingesetzt werden kann:

Hinsichtlich der intuitiven Benutzbarkeit eines Windelverschlussbands wird vorgeschlagen, dass der Rückseitenfarbauftrag an einem Benutzerende des Verschlussbands angeordnet ist. Begrifflich sei hierzu erläutert, dass das Benutzerende dasjenige Ende des Verschlussbandes ist, welchem der Schließbereich benachbart liegt. Mit anderen Worten soll der Farbauftrag gemäß diesem Aspekt in demjenigen Bereich des Verschlussbands liegen, welcher dazu vorgesehen ist, bei an die Windel angebrachtem Verschlussband frei von jedweder permanenter Befestigung am Windelohr zu sein. Dies ist derjenige Bereich des Bandes, welchen der Benutzer ergreifen soll.

In einer bevorzugten Ausführungsform ist der Farbauftrag an einem Fingerlift angeordnet. Es wurde bereits erwähnt, dass der Fingerlift diejenige Stelle am Windelverschlussband präzise verkörpert, an welcher der Benutzer das Band zum Anlegen anfassen soll. Insbesondere liegt der Fingerlift neben der Hakenfläche, so dass während des Greifens des Bandes mit den Fingern die Haken nicht versehentlich überdeckt werden können, was die Verschlusskraft des Bandes schmälern könnte.

Alternativ und kumulativ zu einem rückseitigen Farbauftrag am Fingerlift kann der Farbauftrag im Schließbereich angeordnet sein. Der Schließbereich definiert sich durch denjenigen Bereich, welcher sich als Fläche durch eine Umgrenzende um die Verschlusssystemkomponente ergibt. Bei einer reinen Hakenfläche ist der Schließbereich identisch mit der Hakenfläche. Windelverschlussbänder weisen im Schließbereich mitunter eine Kombination aus Hakenflecken und offener Klebstofflage auf. In einem solchen Fall definiert sich der Schließbereich als Umgrenzende der durch die Haken und den Klebstoff gemeinsam gebildeten Fläche.

Gerade beim Einsatz im Schließbereich können gegenüber bekannten Herstellungsverfahren erhebliche Kosten eingespart werden. So ist ein Farbauftrag im Schließbereich zum einen dadurch möglich, dass die Farbe auf die Verschlusssystemkomponente aufgetragen wird, also beispielsweise auf eine Hakenfläche. Insbesondere ist es aber auch möglich, den Farbauftrag auf einer Unterseite der Haken anzuordnen, also vor allem an einer durchgehenden Kunststoffbasis der Hakenfläche. Darüber hinaus ist es sogar möglich, den Farbauftrag am Fasteningträger unterhalb des Hakenmaterials vorzusehen. Bei geeigneter Werkstoffwahl der Hakenbasis und der Haken selbst scheint ein Farbauftrag zur Rückseite des Bandes hin mehr oder minder durch.

Ein solches Durchscheinen ist bei geeigneter Werkstoffwahl, wie bereits erläutert, zu beiden Seiten des Verschlussbandes hin zu beobachten, also zur Vorderseite und zur Rückseite hin. Zur Vorderseite hin wird jedoch der optische Eindruck durch die Kaschierung der Vorderseite zumindest abgeschwächt. Selbst wenn farblose Fasern verwendet werden, erscheinen diese zur Vorderseite hin wegen der in der Regel verwendeten sehr feinen Fasern meist weißlich, so dass sich zur Vorderseite immer ein verschwommener Eindruck des Drucks auf der Rückseite des Fasteningträgers ergibt. Dies hat sich bei Versuchen des Erfinders allerdings als nicht störend herausgestellt. Vielmehr wird sogar durch das Durchscheinen bereits bei einem Blick des Benutzers auf die Bandvorderseite erkennbar, dass auf der Rückseite eine farbliche Gestaltung vorhanden ist. Dies lenkt die Aufmerksamkeit des Benutzers zu dieser farblichen Gestaltung, wenn er beim An- oder Ablegen oder im abgelegten Zustand die Rückseite des Bandes betrachtet.

Zur Rückseite des Fasteningträgers hin kann der Eindruck relativ klar durchscheinen, selbst wenn er unter den Haken angeordnet ist. Dies hat sich bei zahlreichen Versuchen des Erfinders gezeigt.

Alternativ und kumulativ zur Anordnung am Fingerlift und/oder im Schließbereich kann der Farbauftrag vorteilhaft zwischen dem Schließbereich und dem Befestigungsbereich angeordnet sein. Dieser Abschnitt des Verschlussbands ist hinsichtlich des Schließens der Windel in der Regel nur von untergeordneter Bedeutung. Er ist meist vollflächig elastisch, vollflächig im Wesentlichen inelastisch oder mit elastischen Abschnitten versehen ausgeführt. Dieser Bereich kann jedoch mit einem gezielten Farbauftrag beispielsweise dazu verwendet werden, besondere Informationen an den Benutzer des Verschlussbands weiterzugeben. So kann beispielsweise der Herstellername der Windel aufgedruckt werden, oder es können variierende Informationen bis hin zu rein ästhetischen Gestaltungen, wie beispielsweise verschiedenen Mustern, dort untergebracht werden. Ebenso kann der Farbauftrag im Befestigungsbereich angeordnet werden, wobei dann im Wesentlichen lediglich die durchscheinende Wirkung des Farbauftrages zur Vorderseite hin genutzt wird.

Vorzugsweise weist die Rückseite des Fasteningträgers einen Farbauftrag auf. Ein derartiger Farbauftrag ist in der Produktionslinie verhältnismäßig spät aufzubringen, was die vorgenannten, diesbezüglichen Vorteile insbesondere zur Geltung bringt.

Infolge der eingangs erläuterten unterschiedlichen Autbauvarianten von Windelverschlussbändern kann die Oberfläche auf der Rückseite eines Fasteningträgers unterschiedlich ausgestaltet sein. So kann diese Oberfläche ganz oder zum Teil insbesondere textil - mit oder ohne Befilmung - oder folienartig sein. Demgemäß sei nachstehend zunächst erläutert, wie der Farbauftrag auf die Rückseite besonders vorteilhaft aufgebracht sein kann.

Der Farbauftrag kann beispielsweise auf eine textile Trägeroberfläche oder dessen textile Kaschierung aufgebracht sein. Sowohl der Fasteningträger als solcher als auch eine Trägerkaschierung kann insbesondere ein Nonwoven aufweisen oder vollständig aus Nonwoven bestehen. Wenn auf der Rückseite des Fasteningträgers eine textile Kaschierung aufgebracht wird und diese mit einem Farbauftrag versehen wird, wird das Band sehr komfortabel, weil es auch beim Anlegen auf der Rückseite eine textile Haptik für die Finger des Benutzers bietet. Gleichzeitig sorgt der Farbauftrag für die gewünschte optische Markierung bestimmter Bereiche oder der gesamten Rückseite.

Da ein Farbauftrag unter oder über einer textilen Struktur beim Durchscheinen durch die textile Struktur in der Regel an Schärfe verlieren wird, wird vorgeschlagen, dass ein Farbauftrag auf einer textilen Struktur, sei es eine Kaschierung oder eine textile Bandoberfläche, vollflächig ausgeführt ist. Unter einem vollflächigen Farbauftrag wird verstanden, dass der Farbauftrag eine Fläche vollständig ausfüllt, dass also ein - mit Ausnahme von Begrenzungsrändern - konturloser Auftrag erfolgt. Insbesondere sollen in diesem Fall bevorzugt also nicht spezielle Motive oder Zeichen dargestellt sein.

Alternativ und kumulativ zum Auftrag von Farbe auf eine textile Struktur des Bandes oder seiner Kaschierung wird vorgeschlagen, dass der Farbauftrag auf eine, insbesondere befilmte, Oberfläche des Fasteningträgers aufgebracht ist.

Es wurde bereits erläutert, dass die Grenzen zwischen Band, Kaschierung und Befilmung oft fließend sind. Mit diesem Aspekt soll klargestellt werden, dass der Farbauftrag direkt auf die Oberfläche des Fasteningträgers aufgebracht sein kann, sei diese befilmt oder nicht.

Auf den Fasteningträger direkt kann in der Regel auch relativ scharf konturiert gedruckt werden oder in anderer Weise Farbe aufgetragen werden. So lassen sich diverse Motive und insbesondere auch Schrift problemlos darstellen. Es versteht sich, dass dies bei einer hierfür vorgesehenen Befilmung umso besser der Fall sein kann.

Ein Farbauftrag direkt auf die Oberfläche des Fasteningträgers ist besonders dann sehr präzise ausführbar, wenn diese glatt ist, also folienartig oder stark befilmt. Auch bei einem Farbauftrag auf eine textile Bandrückseitenoberfläche können aber Konturen besser gestaltet werden als auf einer strukturell gleich aufgebauten Vorderseite, weil die Rückseite beim Aufwickeln auf Rollen oder Spulen im Laufe des Herstellprozesses häufig mit einem Liner überdeckt und/oder anderweitig flachgedrückt wird. Infolgedessen liegen textile Strukturen an der Rückseite des Bandes eher flach am Band an als an der Vorderseite. Dies macht sie unter anderem besser bedruckbar.

Insbesondere haben sich bei Versuchen des Erfinders allerdings solche Konstellationen als besonders variabel hinsichtlich verschiedener Farbaufträge herausgestellt, bei welchen der Farbauftrag auf eine mit dem Fasteningträger auf dessen Rückseite verbundene Zusatzlage bzw. auf eine rückseitig der Vorderseite angeordnete Zwischenlage aufgebracht ist. Dies ermöglicht es, die Zusatz- bzw. Zwischenlage, beispielsweise in ihrem Material oder ihrer Oberflächenbeschaffenheit, hinsichtlich des gewünschten Farbauftrags auswählen zu können, während bei der Werkstoffwahl für den Fasteningträger als solchen der Farbauftrag nicht berücksichtigt zu werden braucht.

Wenn eine mit dem Fasteningträger verbundene Zusatzlage vorhanden ist, auf welche die Farbe aufgetragen ist, wird vorgeschlagen, dass die Verschlusssystemkomponente auf die Zusatzlage aufgesetzt ist. Bei einer solchen Gestaltung kann die Zusatzlage relativ großflächig auf den Fasteningträger aufgelegt und mit diesem verbunden werden. Ein Farbauftrag, insbesondere ein Druck, kann sich über eine große Fläche, insbesondere über die vollständige Fläche, der Zusatzlage erstrecken. Das aufgebrachte Motiv oder die aufgebrachte Farbe erscheint in diesem Falle als durchgehender Farbauftrag, so dass die Verschlusssystemkomponente optisch bei geeigneter Gestaltung, insbesondere wenn diese farblos ist, sehr wenig auffällt. So ergibt sich ein optisch sehr gefälliges Verschlussband.

Die Zusatz- bzw. Zwischenlage kann insbesondere eine Folie sein. Eine Folie lässt sich zum einen leicht mit dem Fasteningträger verbinden. Zudem ist sie aufgrund ihrer ausgeprägten Zweidimensionalität bei geeigneter Werkstoffwahl sehr flexibel und gegebenenfalls auch elastisch, so dass der Tragekomfort einer Windel mit einem solchen Verschlussband nicht beeinträchtigt wird. Überdies lässt sich eine Folie günstig beschaffen und leicht mit Farbe auch dauerhaft beaufschlagen, insbesondere bedrucken.

Es wurde bereits erläutert, dass sich insbesondere sehr gefällige optische Eindrücke ergeben, wenn die Verschlusssystemkomponente einen Hakenbereich mit einer transparenten, bevorzugt farblosen, Basis aufweist.

Um die Bänder nochmals variabler gestalten zu können, wird vorgeschlagen, dass der Farbauftrag konturiert und/oder mehrfarbig ist. Hierdurch lassen sich insbesondere graphische Darstellungen aufbringen, im Falle einer Babywindel beispielsweise graphische Darstellungen von Tieren oder geläufigen Gebrauchsgegenständen in Grundfarben. Auch lassen sich Buchstaben und Zahlen ohne weiteres auftragen.

Ergänzend löst die gestellte Aufgabe ein Verschlussband für einen Hygieneartikel, insbesondere für eine Babywindel oder für eine Inkontinenzwindel, mit einem Fasteningträger mit einer textilen oder textil kaschierten Vorderseite und einer auf einer Rückseite des Fasteningträgers in einem Schließbereich getragenen Komponente eines Verschlusssystems zum lösbaren Verbinden mit einer Oberfläche, wobei der Fasteningträger einen elastischen Träger aufweist, der mit Nonwoven kaschiert ist, wobei der Fasteningträger an der dem elastischen Träger zugewandten Seite des Nonwoven einen Farbauftrag mit einer entfernbaren zu der jeweiligen Seite hin wirksamen optischen Überdeckung aufweist.

Begrifflich sei hierzu zunächst erläutert, dass die Überdeckung dergestalt gewählt sein soll, dass ein Durchscheinen und/oder die Erkennbarkeit des Farbauftrags und insbesondere von Konturen des Farbauftrags innerhalb der überdeckten Fläche zu derjenigen Seite hin, an der Farbauftrag und Überdeckung angeordnet sind, weitgehend verhindert werden soll. So können geeignete Überdeckungen aus nahezu beliebigen Werkstoffen mit nahezu beliebiger Farbgebung hergestellt werden. Es ist jedoch von Vorteil, wenn eine Überdeckung zumindest teilweise vollfarbig aufgeführt ist, so dass ein unter der Überdeckung liegender Farbauftrag, beispielsweise ein Druck, durch die Überdeckung von einem menschlichen Auge des Benutzers nicht erkennbar ist.

Neben der Trübung des gewählten Werkstoffs für die Überdeckung spielt auch die gewählte Dicke der Überdeckung eine Rolle. Selbst ein leicht transparenter Werkstoff kann bei ausreichender Dicke den gewünschten Effekt einer unterdrückten Sichtbarkeit bewirken. Insbesondere sei bei einer Überdeckung jedoch an relativ dünne, biegsame Überdeckungen gedacht, die zumindest weitgehend intransparent sind.

Eine Überdeckung selbst kann wiederum einen Farbauftrag aufweisen, beispielsweise einen Aufdruck. Auf diese Weise kann auf der Überdeckung beispielsweise die Information angebracht werden, dass unter der Überdeckung ein weiterer Farbauftrag mit insbesondere weiterer Information vorhanden ist. Auch ist es möglich, an der Überdeckung die eigentliche Information anzubringen, wobei ein mechanisches Bauelement, welches am Fasteningträger angeschlossen ist, zugleich den Farbauftrag und die Überdeckung aufweisen kann.

Ein dergestalt konstruiertes Verschlussband mit einem Farbauftrag und einer entfernbaren optischen Überdeckung kann ohne weiteres dafür eingesetzt werden, Bonuspunkte oder andere Treuemarken am Verschlussband anzubringen. Diese sind bevorzugt durch den eigentlichen Farbauftrag verkörpert. So kann der Farbauftrag eine Zahl mit um sie herum angeordnetem Zierrat sein, wobei die Zahl eine bestimmte Anzahl von Bonuspunkten angibt, welche in einem Treueprogramm des Herstellers der Windeln oder anderer Verbrauchsgüter vergütet wird. Durch die Überdeckung wird in einem solchen Fall verhindert, dass der Käufer frühzeitig den Wert der Bonusmarke unter der Überdeckung erkennt. Selbst wenn dieser immer gleich ist, kann auf diese Weise die Neugier des Benutzers gesteigert werden. Ein wie vorgeschlagen gestaltetes Verschlussband ermöglicht daher zahlreiche technische Ergänzungen auch zu Marketingzwecken.

Beispielsweise können Treuepunkte am Fasteningträger aufgedruckt sein, oder es ist eine abziehbare Folie am Fasteningträger entfernbar gehalten, auf welche die Treuepunkte aufgedruckt sind und welche ihrerseits von einer entfernbar gehaltenen Abdeckfolie überdeckt ist. Der Benutzer des Windelverschlussbands kann in diesem Fall die Abdeckfolie abziehen, abreißen, abschneiden, zur Seite schlagen oder sonstwie entfernen, und er legt auf diese Weise den flächig im Vergleich zur Abdeckfolie kleineren Treuepunkt aus einer leicht aufgeklebten oder beispielsweise rein elektrostatisch gehaltenen oder unbefestigten Folie frei. Der Benutzer kann den Treuepunkt dann abnehmen und aufbewahren, während das Verschlussband - und bei geeigneter Gestaltung auch die Abdeckfolie - an der Windel verbleiben, sodass sie gemeinsam mit dieser entsorgt werden können.

Bei einem Verschlussband mit einem Farbauftrag mit einer entfernbaren optischen Überdeckung wird vorgeschlagen, dass die Überdeckung dergestalt am Farbauftrag angeordnet ist, dass ihre Entfernung irreversibel ist. Eine irreversible Verbindung ist beispielsweise bei einer Perforation gegeben. Wenn also die Überdeckung als Ganzes vom Farbauftrag entfernt werden kann und dabei eine Perforation durchrissen wird, ist es demgemäß nicht mehr möglich, die Überdeckung wie im vorherigen Zustand über dem Farbauftrag wieder anzuordnen.

Eine irreversibel entfernbare Überdeckung bietet den Vorteil, dass sie sicher vom Verschlussband entfernt werden kann. So kann es im Falle einer Babywindel beispielsweise nicht passieren, dass die Überdeckung nach erstmaligem Entfernen durch den Benutzer der Windel versehentlich nur mit einer geringeren Festigkeit wieder angebracht wird. Wenn dies geschähe, bestünde die Gefahr, dass das Baby den Überdeckungsstreifen selbst von der Windel trennt und beispielsweise verschluckt.

Alternativ und kumulativ zu einer irreversiblen Befestigung wird bei einem Verschlussband mit einem Farbauftrag mit entfernbarer Überdeckung vorgeschlagen, dass die Überdeckung mit einer Sollbruchstelle, wie einer Perforation, einem Hotmeltklebepunkt, einer Materialschwächung und ähnlichem, befestigt ist, so dass die Überdeckung von einem Benutzer ohne Abtrag des Farbauftrags entfernt werden kann, zumindest aber ohne wesentlichen Abtrag von Konturen des Farbauftrags. Eine Sollbruchstelle zeichnet sich dadurch aus, dass sie bei der vorhersehbar wahrscheinlichsten Art, wie der Benutzer die Überdeckung abzutrennen versuchen wird, an einer Stelle versagt, den für die zu transportierende Information wesentlichen Farbauftrag nicht beschädigt. In der Regel wird der Benutzer versuchen, an der Überdeckung schlicht zu reißen, sodass sich die Sollbruchstelle bevorzugt durch eine geringe Zugfestigkeit auszeichnen kann. Die Sollbruchstelle kann in einer einfachen Ausführung eine Perforation oder eine schwache Klebeverbindung sein.

Wenn beispielsweise ein Bonussystem mit den Bändern durchgeführt werden soll, ist es wesentlich, dass beim Trennen der Überdeckung, beispielsweise beim Abziehen einer Überdeckungsfolie, der darunter am Band oder an der Überdeckung liegende Informationen tragende Farbauftrag nicht entfernt und bevorzugt nicht einmal beschädigt wird. Es versteht sich, dass gerade beim Auftrag von teils recht filigraner Schrift oder filigranen Mustern der optische Gesamteindruck beim Benutzer gestört würde, wenn beim Entfernen der Überdeckung der Farbauftrag beschädigt würde.

Eine Überdeckungsfolie kann mit einer silikonisierten Zwischenlage angeschlossen sein, um über eine Klebeverbindung angeschlossen zu sein und dennoch leicht abgezogen werden zu können.

Nach einem dritten Aspekt der Erfindung löst die gestellte Aufgabe ein Verschlussband, insbesondere für einen Hygieneartikel wie beispielsweise eine Babywindel oder eine Inkontinenzwindel, mit einem Fasteningträger mit einer textilen oder textil kaschierten Vorderseite und einer auf einer Rückseite des Fasteningträgers in einem Schließbereich getragenen Komponente eines Verschlusssystems zum lösbaren Verbinden mit einer Oberfläche, wobei die Rückseite des Fasteningträgers einen Farbauftrag aufweist.

Ein solches Band, welches nicht zwangsweise als Windelverschlussband eingesetzt werden muss, bringt dem Hersteller des Verschlussbandes sowie dem Weiterverarbeiter des Verschlussbandes die eingangs bereits geschilderten Vorteile größerer Flexibilität in der Herstellung. Selbst bei einem Windelverschlussband muss in der Fertigungsstufe beim Bandhersteller nicht notwendigerweise der Befestigungsbereich bereits vorbereitet sein.

Nach einem vierten Aspekt der Erfindung löst die gestellte Aufgabe ein Bandmaterial mit einer Längserstreckungsrichtung, welches durch Trennen quer zur Längserstreckungsrichtung und gegebenenfalls auch im wesentlichen in Längserstreckungsrichtung zu Einzelnutzen in Form von Verschlussbändern, bevorzugt ohne Verschnitt, aufteilbar ist, und zwar bevorzugt in Form einer Wicklung, vor allem einer Rolle oder einer Spule. Das Bandmaterial kann in dieser Form leicht gehändelt und vom Bandhersteller zum Weiterverarbeiter transportiert werden. Auch kann es beim Weiterverarbeiter, beispielsweise einem Windelhersteller, in einer solchen Form in eine entsprechende Ansetzmaschine in Maschinenrichtung eingeführt werden.

Es versteht sich, dass die durch das Band erreichten Vorteile auch an einer Windel feststellbar sind, welche mit einem wie vorstehend geschildertem Verschlussband ausgestattet ist.

Nach einem fünften Aspekt der Erfindung löst die Aufgabe ein Verfahren zum Herstellen verschieden auf der Rückseite mit Farbauftrag versehener Verschlussbänder oder zum Herstellen verschieden auf der Rückseite mit Farbauftrag versehener Bandmateriale, wobei die Verschlussbänder mit einheitlichen mechanischen Elementen ausgestattet werden, die teilweise verschieden bedruckt werden.

Es wurde bereits eingangs erläutert, dass der Bandhersteller und der Windelhersteller beim Vorsehen eines Farbauftrags auf die Rückseite des Verschlussbandes eine höhere Flexibilität der Farbgestaltung haben. Das nun vorgestellte Verfahren nutzt diese Vorteile in besonderem Maße für sich aus.

In einer bevorzugten Ausgestaltung des Verfahrens wird ein Windelverschlussband zunächst so weit fertig gestellt, dass es mechanisch vollständig ist, und anschließend wird das Windelverschlussband auf seiner Rückseite unmittelbar oder mittelbar mit einem Farbauftrag versehen, und zwar bevor, während oder nachdem das Windelverschlussverband an der Windel befestigt wird.

Bei einer solchen Vorgehensweise wird das Windelverschlussband zunächst als solches mechanisch fertig gestellt. Auf den Fasteningträger wird also auf der Vorderseite die Kaschierung aufgebracht, oder es wird ein Fasteningträger verwendet, welcher bereits eine textile Vorderseite aufweist; auf der Rückseite wird der Schließbereich eingerichtet, beispielsweise indem auf der Rückseite des Fasteningträgers eine Vielzahl von Haken mit einer gemeinsamen Hakenbasis oder in Form getrennter Streifen über eine Klebstoffschicht befestigt wird. Auch können weitere mechanische Elemente vorgesehen werden, beispielsweise eine textile Kaschierung auf einem Teil der Rückseite oder eine Folie auf der Rückseite.

In dieser Form ist das Windelverschlussband bis auf den Farbauftrag fertig gestellt, so dass es beispielsweise in dieser Form auch auf Lager vorgehalten werden kann. Wenn dann von einem oder mehreren Abnehmern Lieferaufträge eingehen, müssen die mechanisch bereits fertig gestellten Windelverschlussbänder nur noch mit dem Farbauftrag versehen werden. Hierzu können sie beispielsweise bedruckt werden, was sehr schnell geht und auch kleine Chargen wirtschaftlich ermöglicht.

Andererseits können diese Vorteile auch bei Zwischenlagerung von Halbzeugen, die beispielsweise für Zusatz- oder Zwischenlagen genutzt werden, zur Geltung kommen. Ebenso kann jedoch, beispielsweise bei einer Inline-Fertigung der Farbauftrag während eines Laminierens bzw. zusammenfügen verschiedener Lagen aufgebracht werden.

Alternativ können die Bänder auch ohne Farbauftrag zum Weiterverarbeiter gesendet werden, beispielsweise zum Windelhersteller. Der Windelhersteller kann die Bänder in diesem Falle selbst mit Farbauftrag versehen, beispielsweise bedrucken. Dies kann in einem vorgeschalteten Arbeitsgang zum Befestigen an der Windel geschehen. Ebenso ist es aber denkbar, dass die Ansetzmaschine um eine Druckeinheit erweitert wird, so dass der Farbauftrag am Bandmaterial oder an den zerschnittenen Einzelnutzen in einem Arbeitsgang mit dem Befestigen an der Windel erfolgen kann.

Auch ist es denkbar, dass der Windelhersteller die Windeln bereits vollständig mit den Verschlussbändern versieht und erst danach den Farbauftrag anbringt. Bei einem solchen Vorgehen behält sich der Windelhersteller eine größtmögliche Produktionsflexibilität bis kurz vor Auslieferung der Windeln vor.

Die Erfindung wird nachfolgend anhand verschiedener Ausführungsbeispiele unter Bezugnahme auf die Zeichnung näher erläutert. Es zeigen:
- Fig. 1: zur Erläuterung des erfinderischen Umfeldes in einem schematischen Schnitt ein Bandmaterial mit einem bedruckten Folienstreifen auf einer Rückseite eines Fasteningträgers,
- Fig. 2: zur Erläuterung des erfinderischen Umfeldes in einer schematischen per- spektivischen Ansicht einen schmalen Streifen des Bandmaterials aus Fig. 1,
- Fig. 3: zur Erläuterung des erfinderischen Umfeldes in einem schematischen Schnitt einen Einzelnutzen aus dem Bandmaterial aus den Figuren 1 und 2,
- Fig. 4: zur Erläuterung des erfinderischen Umfeldes in einer schematischen Draufsicht einen Bandmaterialstreifen ähnlich demjenigen aus Fig. 2 mit einer alternativen Fingerlift-Schnittlinie,
- Fig. 5: zur Erläuterung des erfinderischen Umfeldes in einem schematischen Schnitt analog demjenigen aus Fig. 1 eine zweite Variante eines Bandma- terials mit einem bedruckten Folienstreifen, welcher sich zwischen zwei Befestigungsbereichen erstreckt,
- Fig. 6: zur Erläuterung des erfinderischen Umfeldes in einem analogen Schnitt eine dritte Variante eines Bandmaterials mit einem Druck unter einer Hakenbasis,
- Fig. 7: zur Erläuterung des erfinderischen Umfeldes in einem weiteren analogen Schnitt eine vierte Variante eines Bandmaterials mit einer bedruckten Fo- lie, welche sich auf einer Rückseite eines Fasteningträgers über dessen gesamte Breite erstreckt,
- Fig. 8: zur Erläuterung des erfinderischen Umfeldes in einem weiteren analogen Schnitt eine fünfte Variante eines Bandmaterials mit einer bedruckten Folie und bedruckter Hakenbasis,
- Fig. 9: zur Erläuterung des erfinderischen Umfeldes in einem weiteren analogen Schnitt eine sechste Variante eines Bandmaterials mit innerhalb des Nonwovens vorgesehenem Druck,
- Fig. 10: zur Erläuterung des erfinderischen Umfeldes in einem weiteren analogen Schnitt eine siebte Variante eines Bandmaterials mit innerhalb des Non- wovens vorgesehenem Druck,
- Fig. 11: in einem weiteren analogen Schnitt eine achte erfindungsgemäße Varian- te eines Bandmaterials mit innerhalb des Nonwovens vorgesehenem Druck,
- Fig. 12: in einem weiteren analogen Schnitt eine neunte erfindungsgemäße Vari- ante eines Bandmaterials mit innerhalb des Nonwovens vorgesehenem Druck,
- Fig. 13: zur Erläuterung des erfinderischen Umfeldes in einem weiteren analogen Schnitt eine zehnte Variante eines Bandmaterials mit innerhalb des Non- wovens vorgesehener Überdeckung,
- Fig. 14: zur Erläuterung des erfinderischen Umfeldes in einem weiteren analogen Schnitt eine elfte Variante eines Bandmaterials mit auf der Rückseite des Nonwovens vorgesehener Überdeckung,
- Fig. 15: zur Erläuterung des erfinderischen Umfeldes in einem weiteren analogen Schnitt eine zwölfte Variante eines Bandmaterials mit auf einer Träger- schicht unter einer Überdeckung vorgesehenem Druck,
- Fig. 16: zur Erläuterung des erfinderischen Umfeldes in einer weiteren zu Figur 3 analogen Darstellung ein Windelohr und
- Fig. 17: zur Erläuterung des erfinderischen Umfeldes in einer weiteren zu Figur 3 analogen Darstellung ein weiteres Windelohr mit einem Verschlussband.

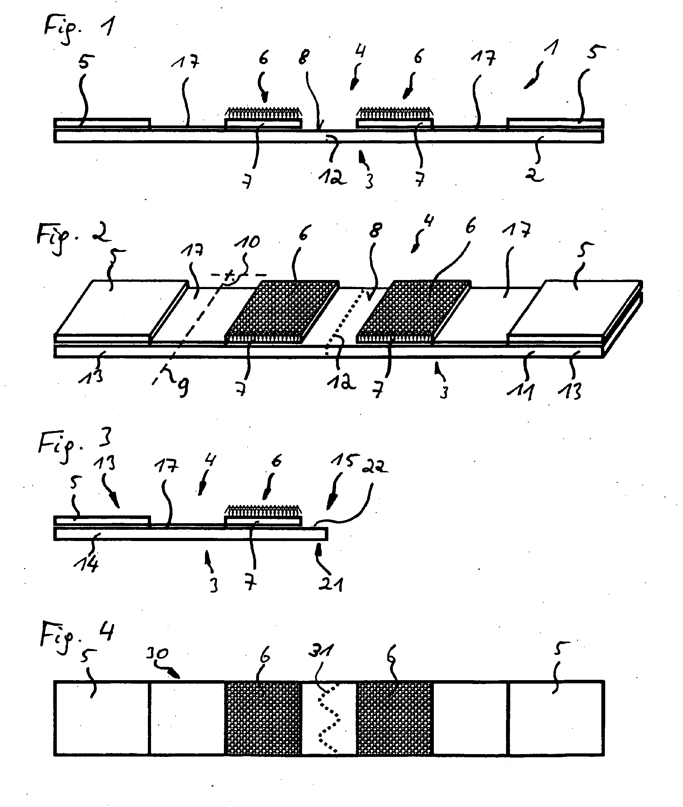

Das Bandmaterial 1 in Fig. 1 besteht aus einem Endlosband 2 eines eigenstabilen Nonwoven mit einer Vorderseite 3 und mit einer befilmten (nicht explizit dargestellt) Rückseite 4.

Es versteht sich, dass ebenso ein Endlosband mit anderer mechanischer Struktur als Fasteningträger eingesetzt werden könnte. Insbesondere sei hier an ein Nonwoven mit einer unbefilmten Rückseite 4 gedacht, oder an ein Laminat mit einer textilen Lage zur Vorderseite 3 hin und mit einer Folie oder einem Film zur Rückseite 4 hin. Ebenso kann ein Laminat, welches eine elastische Lage oder Teillage umfasst, vorteilhaft entsprechend zur Anwendung kommen.

Auf der Rückseite 4 ist das Endlosband 2 in symmetrischem Aufbau mit einem Permanentklebstoff 5 und einem Hakenmaterial 6 ausgerüstet. Das Hakenmaterial 6 besteht aus einer Vielzahl von Einzelhaken oder Einzelpilzköpfen, welche aus Kunststoff geformt sind und in eine gemeinsame Kunststoffbasis 7 übergehen. Die Kunststoffbasis 7 ist über einen Permanentklebstoff (nicht dargestellt) auf eine Oberfläche 8 des Endlosbandes 2 aufgeklebt.

Das Bandmaterial 1 ist dazu vorgesehen, eine Maschine in einer Maschinenrichtung 9 (dargestellt in Fig. 2) zu durchlaufen und während des Durchlaufs in cross-direction 10 senkrecht zur Maschinenlaufrichtung 9 zu Bandstreifen 11 durchtrennt zu werden. Die Bandstreifen 11 sind dazu vorgesehen, durch einen Schnitt entlang einer Schnittlinie 12 zu Einzelnutzen 13 in Form jeweils eines vollständigen Windelverschlussbands durchtrennt zu werden. Die Einzelnutzen 13 können über den Permanentklebstoff 5 permanent am Windelohr einer Windel angesetzt werden. Das durchtrennte Endlosbandmaterial 2 wird auf diese Weise sicher am Windelohr fixiert und dient dem einzelnen Windelverschlussband 13 als Fasteningträger 14.

An einem in diesem Befestigungszustand freien Benutzerende 15 des Windelverschlussbands 13 ist das Hakenmaterial 6 angeordnet und dient dazu, die Windel während des Tragens und/oder zur Entsorgung sicher öffenbar zu verschließen.

Zwischen dem Hakenmaterial 6, welches durch seine Umgrenzung den Schließbereich des Windelverschlussbands definiert, bzw. neben dessen Basis 7, und dem Permanentklebstoff 5, welcher durch seine Umgrenzung den Befestigungsbereich des Windelverschlussbands definiert, ist die befilmte Rückseite des Nonwovens 2 in einem Bereich 17 bedruckt. In einer alternativen Ausführungsform kann hier auch eine bedruckte Folie aufgeklebt werden. Die bedruckten Bereiche 17 werden beim Durchtrennen in cross-direction 10 und in Maschinenlaufrichtung 9 entlang der Schnittlinie 12 ebenfalls zerschnitten, so dass sie an dem Windelverschlussband 13 entsprechend vorliegen.

Der bedruckte Bereich 17 ist am Windelverschlussband 13 neben dem Befestigungsbereich angeordnet, so dass es für den Benutzer der Windel, beispielsweise ein Elternteil beim Anlegen einer Babywindel an das Baby, sichtbar ist. Auf dem bedruckten Bereich 20 können Informationen für den Benutzer untergebracht werden, beispielsweise durch einen Textaufdruck, oder es können graphische Gestaltungen oder durchgehende Farbflächen dort aufgetragen sein.

Alternativ und kumulativ zum bedruckten Bereich 20 kann auch im frei überstehenden Bereich 21 des Fasteningträgers 14, üblicherweise als Benutzerende 15 bezeichnet, an einem Fingerlift (nicht dargestellt, mögliche Position auf der Bandrückseite jedoch dargestellt mit der Bezugsziffer 22) ein Folienstück mit einer Bedruckung oder einem anderen Farbauftrag bzw. ein entsprechender bedruckter Bereich vorgesehen sein. Anstelle des Folienstücks oder zusätzlich zum Folienstück kann dort beispielsweise auch eine textile Struktur mit einem Auftrag vorgesehen sein. So sei insbesondere an eine am Benutzerende lokale textile Kaschierung einer Folienoberfläche oder auch an eine textile Rückseite des Fasteningträgers gedacht.

Bei geeigneter Werkstoffwahl eines bedruckten Folienstreifens kann ein Aufdruck auf diesen gegen mechanischen Abrieb geschützt werden, wenn der Aufdruck nicht auf der vom Fasteningträger abgewandten Oberfläche 17 angeordnet ist, sondern auf einer zum Fasteningträger gerichteten unteren Oberfläche 23. Wenn der Folienstreifen selbst aus einem transparenten Material hergestellt ist, erscheint der Aufdruck zur Rückseite 4 des Windelverschlussbands 13 hin dennoch in unbeeinträchtiger Schärfe.

Bei Versuchen des Erfinders haben sich weitgehend unabhängig von der Transparenz des für den Folienstreifen gewählten Werkstoffs Banddicken zwischen 10 µm und 50 µm als vorteilhaft herausgestellt. Besonders vorteilhafte Ergebnisse erzielten bedruckte Folien mit einer Dicke von etwa 20 µm auf einem Fasteningträger aus thermoplastischem Elastomer mit einer Flächenmasse zwischen 300 g/m² und 400 g/m².

Der Bandstreifen 30 in Fig. 4 ist im mechanischen Aufbau mit demjenigen aus Fig. 2 identisch. Beim Durchtrennen zu zwei Einzelnutzen ist jedoch eine mäandrierende Schnittlinie 31 vorgesehen.

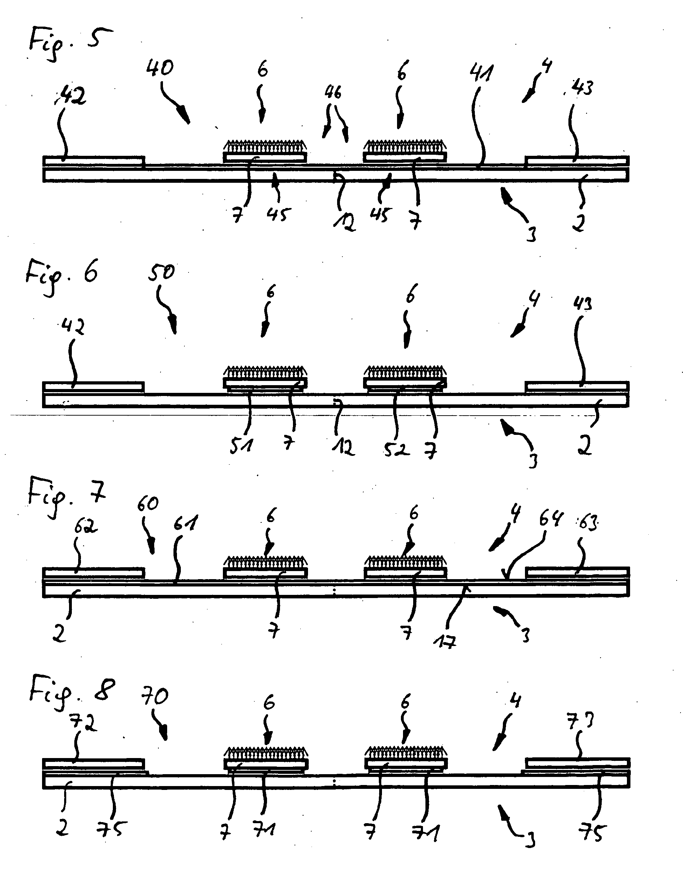

Die zweite Variante 40 in Fig. 5 eines Bandmaterials unterscheidet sich vom Bandmaterial 1 aus Fig. 1 darin, dass ein breiter bedruckter Bereich 41 zwischen den beiden als Befestigungsbereich dienenden Klebstoffflächen 42, 43 vorgesehen ist. Somit erfolgt ein Druck auch in dem überstehenden Bereich 46, der als Fingerlift genutzt werden kann, während die Befestigung der Hakenbereiche 6 in gleicher Weise wie beim Bandmaterial 1 erfolgt.

Durch gezieltes Bedrucken des Bereichs 41 können die später entstehenden Einzelnutzen nicht nur im Bereich 44 zwischen dem Permanentbereich und dem Schließbereich auf flexible Weise mit einer Farbgebung oder Konturen versehen werden. Vielmehr erstreckt sich dieser Vorteil nun auch auf einen Bereich 45 unter der Hakenbasis 7 und einen Bereich 46 am freien Ende 21 des Windelverschlussbands. Diese Teilbereiche können sogar unterschiedlich hinsichtlich des Farbauftrags gestaltet werden. So kann beispielsweise im Zwischenbereich 44 eine Information, wie beispielsweise eine Bonuspunktinformation, untergebracht werden, während der Schließbereich durch eine durchscheinende Farbgebung in einer Signalfarbe gekennzeichnet wird und ein Fingerlift am Überstandsbereich 46 ebenfalls in einer Signalfarbe gekennzeichnet wird.

Insbesondere bei Babywindeln ist auch ohne weiteres denkbar, eine durchgehende graphische Gestaltung mit Tieren, Gebrauchsgegenständen und einfacher Farbgebung vorzusehen. Wenn die Basis 7 des Hakenmaterials 6 hochtransparent gestaltet ist, ist eine graphische Gestaltung im Bereich 45 unter der Hakenbasis 7 zur Rückseite 4 des Bandes hin für einen Benutzer sehr deutlich erkennbar. Gleichzeitig kann bei geeigneter Wahl der Kaschierung des Bandmaterials 2 bzw. bei geeigneter Wahl eines textilen Bandmaterials auf der Vorderseite 3 eine Farbgebung oder ein Motiv durchschimmern.

Bei der dritten Variante 50 eines Bandmaterials in Fig. 6 sind wiederum zwei bedruckte Bereiche 51, 52 vorgesehen, in dem ein Farbauftrag unmittelbar an der Basis 7 der Haken 6 erfolgt ist. Diese Bereiche 51, 52 erstrecken sich allerdings nur unterhalb der Hakenflächen und dienen als Befestigungsbasis für die Hakenbasis 7. Es versteht sich, dass stattdessen auch eine bedruckte Zusatz- oder Zwischenlage zur Anwendung kommen kann.

Bei der vierten Variante 60 eines Bandmaterials in Fig. 7 ist ein breiter Folienstreifen 61 mit einem Farbauftrag 17 auf dem Endlosband 2 befestigt. Dieser erstreckt sich über die gesamte Breite des Bandmaterials 2 und ist auf der Vorderseite des Folienstreifens 61 angebracht. Somit sind die Klebstoffflächen 62, 63 zum permanenten Befestigen am Windelohr auf der freien Oberfläche 64 des bedruckten Folienstreifens 61 angeordnet. Dadurch, dass der Folienstreifen 61 auf seiner Vorderseite noch mit dem Nonwoven 2 kaschiert ist, handelt es sich in Bezug auf den gesamten Fasteningträger dennoch um einen rückseitigen Farbauftrag.

Es sei darauf hingewiesen, dass der Fasteningträger für ein Windelverschlussband in der Regel auf seiner Rückseite befilmt ist, wenn er zur Rückseite hin eine textil strukturierte Oberfläche aufweist. Eine solche Befilmung ist zum Anwenden der Erfindung weder erforderlich noch hinderlich. Sie kann förderlich sein, da auf die Befilmung bei geeigneter Werkstoffwahl besser beispielsweise gedruckt werden kann als auf die Filamente oder Fasern ohne die Befilmung.

Es sei auch nochmals darauf hingewiesen, dass es auch Bandkonstruktionen gibt, bei welchen statt eines befilmten Fasteningträgers auf eine Textillage zur Rückseite des Bandes hin eine Folie autkaschiert wird. Ein solcher Aufbau ist insbesondere bei Windelverschlussbändern anzutreffen, welche im Auslieferungszustand an den Endkunden vorgeschnitten sind und sich durch die Bildung elastischer Streifen und inelastischer Streifen insgesamt elastisch verhalten. Es versteht sich, dass auch bei dergestalt aufgebauten Verschlussbändern die Erfindung ohne weiteres angewendet werden kann. Es wurde eingangs bereits erläutert, dass die Abgrenzung zwischen den verschiedenen Bandtypen, die in der vorliegenden Anmeldung behandelt werden, oft fließend ist.

Die Ausführungsform 70 nach Figur 8 stellt eine Kombination zwischen den in Figuren 6 und 7 dargestellten Ausführungsformen dar. Hierbei ist einerseits die Basis 7 der Haken 6 in Bereichen 71 und andererseits eine Zwischenlage 75 unter den Klebstoffflächen 72, 73 bedruckt, welche darüber hinaus eine Migration des Klebstoffs in das Nonwoven 2 verhindert, so dass insbesondere bei dieser Ausführungsform auf eine Befilmung verzichtet werden kann.

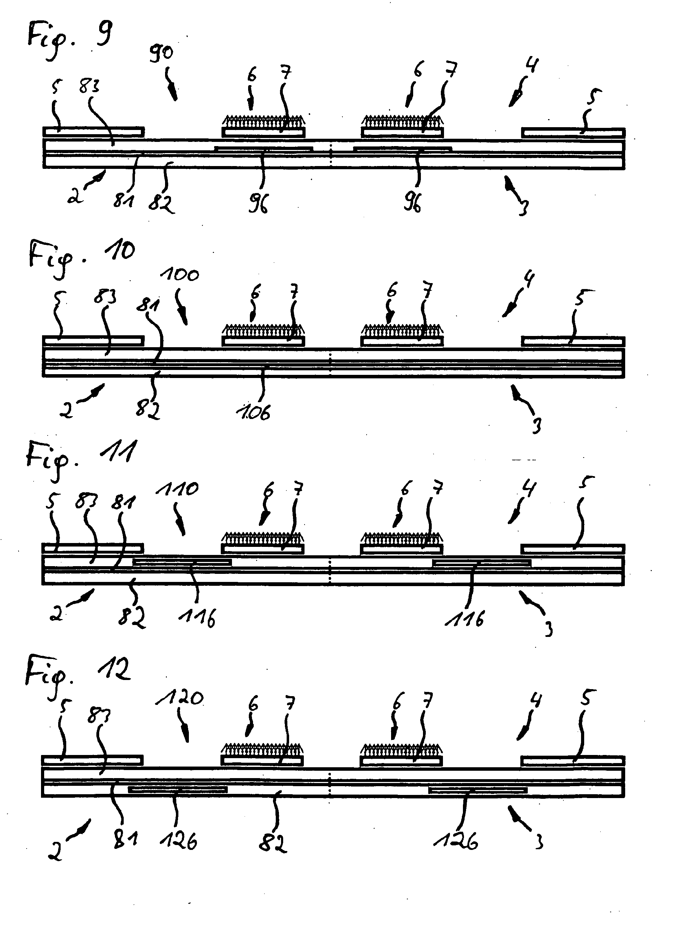

Die in den Figuren 9 bis 12 dargestellten Bandmaterialien 90 bis 120 sind in ihrem Aufbau, insbesondere der Trägerlage an sich, etwas komplexer. So weist die Nonwovenlage 2 jeweils einen elastischen Träger 81 auf, der vorder- und rückseitig mit einem an sich nicht eigenstabilen Nonwoven 82, 83 kaschiert ist. Insgesamt wird hierdurch ein elastischer und eigenstabiler Nonwovenverbund geschaffen, der, je nach konkreter Herstellungsweise, zunächst aktiviert werden muss, bis er seine Elastizität voll entfaltet. Ebenso kann dieser Verbund durch die Basis 7 der Haken 6 bzw. durch seine Befestigung an einer Windel oder einem ähnlichen Gegenstand in den Bereichen 5 in seiner Elastizität gehemmt werden.

Bei dem Ausführungsbeispiel nach Figur 9 ist hierbei im Bereich der Haken 6 der elastische Träger 81 rückseitig (4) in einem Bereich 96 bedruckt. Hiervon abweichend weist das Ausführungsbeispiel nach Figur 10 vorderseitig (3) einen durchgehenden Druck 106 auf, welcher von der Nonwovenlage 82 vorderseitig (3) abgedeckt und somit rückseitig der vorderen Nonwovenlage 82 angeordnet ist. Bei den Ausführungsbeispielen nach Figuren 11 und 12 ist jeweils das Nonwoven 82 bzw. 83 bedruckt, wobei der Druck 116, 126 jeweils auf der dem elastischen Träger zugewandten Seite erfolgt ist. Ein derartiger Druck kann auch bei einem an sich nicht eigenstabilen Nonwoven erfolgen, unmittelbar bevor dieses stabilisiert, beispielsweise auf den elastischen Träger 81 aufgebracht, wird.

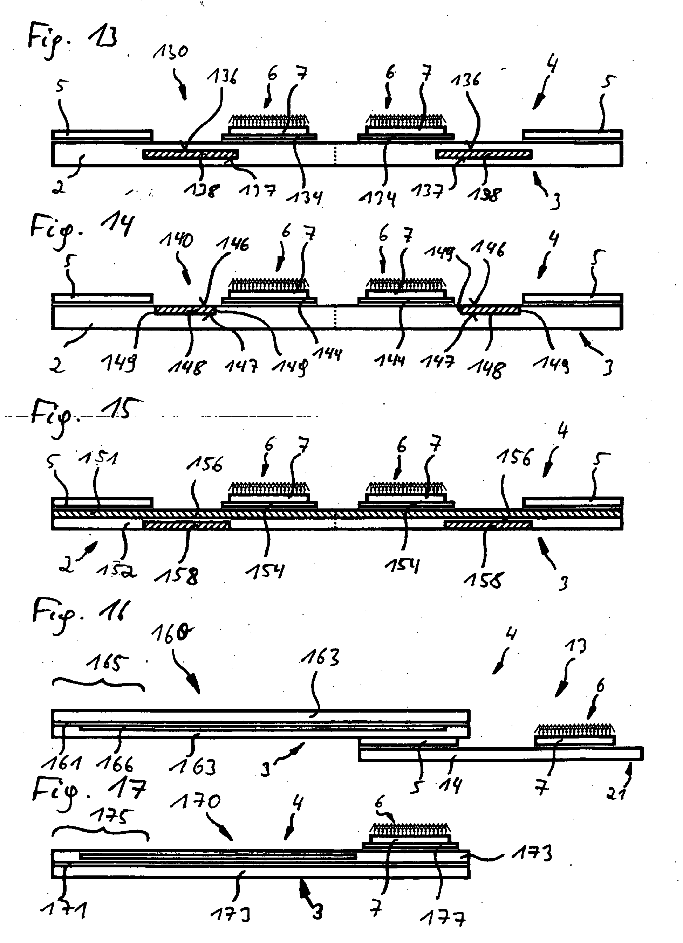

Die in den Figuren 13 und 14 dargestellten Bandmaterialien 130, 140 weisen jeweils eine bedruckte Zusatzlage 134, 144 sowie Überdeckungen 138, 148 auf, die in das Nonwoven 2 eingebracht sind. Die Überdeckungen 138, 148 sind jeweils in Bereichen 136, 137 und 146, 147 bedruckt und können in abgewandelten Ausführungsformen auch noch andere Drucke abdecken. Es versteht sich, dass derartige Überdeckungen mit sämtlichen hier dargestellten Ausführungsformen zur Anwendung kommen und beispielsweise aus stärkerem Folienmaterial, insbesondere aus einzelnen Folien- oder Kunststoffplättchen, gebildet sein können. Die Überdeckungen 138, 148 können aus bzw. von dem Nonwoven 2 entfernt werden. Hierbei geht, wie unmittelbar ersichtlich, bei dem Ausführungsbeispiel nach Figur 13 die strukturelle Integrität des Bandes verloren, während bei dem Ausführungsbeispiel nach Figur 14 die Überdeckung 148 über eine Perforation 149 mit dem Nonwoven 2 verbunden ist, so dass die Überdeckung 148 ohne Verlust der Integrität entfernt werden kann.

Letzteres gilt auch für das Ausführungsbeispiel nach Figur 15, bei welcher ein elastischer Träger 151 ein an sich eigenstabiles Nonwoven 152 trägt, in welches eine Überdeckung 158 eingebettet ist. Nach Entfernen der Überdeckung 158 wird ein Druck 156 freigelegt, der auf dem Träger 151 aufgebracht ist. Gleichzeitig wird auf diese Weise das elastische Material derart befreit, dass es gedehnt werden kann, da zwischen der Überdeckung 158 und dem Träger 151 lediglich eine sehr dünne und mithin nicht sehr stabile Schicht Nonwoven 152 verbleibt. Auch diese Ausfühmngsbeispiel umfasst eine bedruckte Zwischenlage 154 zwischen Basis 7 der Haken 6 und dem elastischen Träger 151.

In den Figuren 16 und 17 sind jeweils Windelohren beschrieben, die in ihrem prinzipiellen Aufbau den vorstehend beschriebenen Verschlussbändern, insbesondere denen nach Figuren 9 bis 12, entsprechen und mithin dadurch, dass sie Verschlussmittel, nämlich Haken 6, tragen, dementsprechend Verschlussbänder 160, 170 darstellen, die in Bereichen 165, 175 an einer Windel oder ähnlichen befestigt werden können. Gegebenfalls können hier Klebestreifen, ähnlich wie die Klebestreifen 5, 42, 43, 62, 63 zur Anwendung kommen. Auch diese Windelohren 160, 170 weisen einen elastischen Träger 161, 171 auf. Dieser ist bei dem Ausführungsbeispiel nach Figur 16 mit einem Druck 166 versehen und beidseits mit Nonwoven 163 kaschiert. Bei dem Ausführungsbeispiel nach Figur 16 ist darüber hinaus auf der Vorderseite des Nonwovens 163 ein Verschlussband 13 ähnlich dem Verschlussband nach Figur 3 angebracht, so dass diesbezüglich auf eine Detailerläuterung verzichtet und identische Bezugsziffern verwendet werden. Es versteht sich, dass auch dieses Verschlussband 13 erfindungsgemäß ausgestaltet sein kann, was jedoch nicht zwingend notwendig ist. Das Ausführungsbeispiel nach Figur 17 verzichtet hingegen auf ein separates Verschlussband, so dass die Haken 6 unmittelbar mit ihrer Basis 7 auf der Rückseite des Nonwovens 173 angeordnet sind. Hierbei ist zwischen der Basis 7 und dem Nonwoven 173 noch eine bedruckte Folie 177 angeordnet. Es ist unmittelbar ersichtlich, dass derartige Windelohren in den verschiedensten Ausgestaltungen, insbesondere ähnlich wie die in den Figuren 1 bis 15 dargestellten kleineren Verschlussbänder, variiert werden können, um die Erfindung umzusetzen.

## Patentansprüche

1. Verschlussband (13) mit einem Fasteningträger (14) mit einer textilen oder textil kaschierten Vorderseite (3) und einer auf einer Rückseite (4) des Fasteningträgers (14) in einem Schließbereich getragenen Komponente (6) eines Verschlusssystems zum lösbaren Verbinden mit einer Oberfläche, wobei der Fasteningträger einen elastischen Träger (81, 161, 171) aufweist, der mit Nonwoven (82, 83, 163, 173) kaschiert ist, ***gekennzeichnet durch*** einen Farbauftrag (17) auf der dem elastischen Träger (81) zugewandten Seite des Nonwoven (82, 83, 163, 173).

2. Verschlussband nach Anspruch 1, ***dadurch gekennzeichnet, dass*** der Fasteningträger an der dem elastischen Träger (81, 161, 171) zugewandten Seite des Nonwoven (82, 83, 163, 173) einen Farbauftrag mit einer entfernbaren zu der jeweiligen Seite hin wirksamen optischen Überdeckung (138, 148, 158) aufweist.

3. Verschlussband nach Anspruch 2, ***dadurch gekennzeichnet, dass*** die Überdeckung (138, 148, 158) irreversibel entfernbar ist.

4. Verschlussband nach Anspruch 2 oder 3, ***dadurch gekennzeichnet, dass*** die Überdeckung (138, 148, 158) mit einer Sollbruchstelle (149) befestigt ist, sodass die Überdeckung von einem Benutzer ohne Abtrag des Farbauftrags entfernt werden kann, zumindest aber ohne wesentlichen Abtrag von Konturen des Farbauftrags.

5. Verschlussband nach einem der Ansprüche 2 bis 4, ***dadurch gekennzeichnet, dass*** die Überdeckung (138, 148, 158) einen Farbauftrag aufweist.

6. Verschlussband nach einem der vorstehenden Ansprüche**,** ***dadurch gekennzeichnet, dass*** der elastische Träger (81, 161, 171) vorder- und rückseitig mit Nonwoven (82, 83, 163, 173) kaschiert ist.

7. Verschlussband nach einem der vorstehenden Ansprüche, ***dadurch gekennzeichnet, dass*** der Farbauftrag ein Aufdruck ist.

8. Verschlussband einem der vorstehenden Ansprüche, ***dadurch gekennzeichnet, dass*** der Farbauftrag (17) an einem Benutzerende (15) des Verschlussbands angeordnet ist.

9. Verschlussband nach einem der vorhergehenden Ansprüche, ***dadurch gekennzeichnet, dass*** der Farbauftrag (17) an einem Fingerlift (22) angeordnet ist.

10. Verschlussband nach einem der vorhergehenden Ansprüche, ***dadurch gekennzeichnet, dass*** der Farbauftrag (41, 52, 61) im Schließbereich (45) angeordnet ist.

11. Verschlussband nach einem der vorhergehenden Ansprüche, ***dadurch gekennzeichnet, dass*** der Farbauftrag zwischen dem Schließbereich (45) und dem Befestigungsbereich (5, 42, 43) angeordnet ist.

12. Verschlussband nach einem der vorhergehenden Ansprüche, ***dadurch gekennzeichnet, dass*** die Rückseite (4) des Fasteningträgers (14) einen Farbauftrag (17) aufweist.

13. Verschlussband nach einem der vorhergehenden Ansprüche, ***dadurch gekennzeichnet, dass*** der Farbauftrag auf ein an sich nicht eigenstabiles Nonwoven (82, 83, 163, 173) aufgebracht ist.

14. Verschlussband nach einem der vorhergehenden Ansprüche, ***dadurch gekennzeichnet, dass*** eine textile Struktur der Bandoberfläche bzw. der Kaschierung den Farbauftrag vollflächig aufweist.

15. Verschlussband nach einem der vorhergehenden Ansprüche, ***dadurch gekennzeichnet, dass*** der Farbauftrag auf eine Oberfläche des Fasteningträgers aufgebracht ist.

16. Verschlussband nach einem der vorhergehenden Ansprüche, ***dadurch gekennzeichnet, dass*** der Farbauftrag auf eine mit dem Fasteningträger (14) verbundene Zusatz- und/oder Zwischenlage aufgebracht ist.

17. Verschlussband nach Anspruch 16, ***dadurch gekennzeichnet, dass*** der Farbauftrag auf eine Zusatzlage aufgebracht ist und die Verschlusssystemkomponente (6, 7) auf die Zusatzlage (41, 52, 61) aufgesetzt ist.

18. Verschlussband nach Anspruch 16 oder 17, **dadurch gekennzeichnet, dass** die Zusatz- und/oder Zwischenlage eine Folie aufweist.

19. Verschlussband nach einem der vorhergehenden Ansprüche, ***dadurch gekennzeichnet, dass*** die Verschlusssystemkomponente einen Hakenbereich mit einer transparenten Basis aufweist.

20. Verschlussband nach einem der vorhergehenden Ansprüche, ***dadurch gekennzeichnet, dass*** der Farbauftrag konturiert und/oder mehrfarbig ist.

21. Bandmaterial (2) mit einer Längserstreckungsrichtung (9), welches durch Trennen quer zur Längserstreckungsrichtung (9), und gegebenenfalls auch im wesentlichen in Längserstreckungsrichtung, zu Einzelnutzen (13) in Form von Verschlussbändern nach einem der vorhergehenden Ansprüche aufteilbar ist.

22. Windel mit einem Verschlussband nach einem der Ansprüche 1 bis 20.

## Claims

1. A sealing tape (13) comprising a fastening carrier (14) with a textile or textile-laminated front side (3) and a component (6) of a sealing system carried on a rear side (4) of the fastening carrier (14) in a closure region for detachable connection to a surface, wherein the fastening carrier comprises an elastic carrier (81, 161, 171) which is laminated with nonwovens (82, 83, 163, 173), **characterised by** a colour application (17) on the side of the nonwoven (82, 83, 163, 173) facing the elastic carrier (81).

2. The sealing tape according to claim 1, **characterised in that** on the side of the nonwoven (82, 83, 163, 173) facing the elastic carrier (81, 161, 171), the fastening carrier has a colour application with an effective visual covering (138, 148, 158) removable towards the respective side.

3. The sealing tape according to claim 2, **characterised in that** the covering (138, 148, 158) is irreversibly removable.

4. The sealing tape according to claim 2 or 3, **characterised in that** the covering (138, 148, 158) is fastened with a predetermined breaking point (149) so that the covering can be removed by a user without removing the colour application, but at least without substantial removal of contours of the colour application.

5. The sealing tape according to any one of claims 3 to 4, **characterised in that** the covering (138, 148, 158) has a colour application.

6. The sealing tape according to any one of the preceding claims, **characterised in that** the elastic carrier (81, 161, 171) is laminated with nonwoven (82, 83, 163, 173) on the front and rear side.

7. The sealing tape according to any one of the preceding claims, **characterised in that** the colour application is an imprint.

8. The sealing tape according to any one of the preceding claims, **characterised in that** the colour application (17) is disposed at a user end (15) of the sealing tape.

9. The sealing tape according to any one of the preceding claims, **characterised in that** the colour application (17) is disposed on a finger lift (22).

10. The sealing tape according to any one of the preceding claims, **characterised in that** the colour application (41, 52, 61) is disposed in the closure region (45).

11. The sealing tape according to any one of the preceding claims, **characterised in that** the colour application is disposed between the closure region (45) and the fastening region (5, 42, 43).

12. The sealing tape according to any one of the preceding claims, **characterised in that** the rear side (4) of the fastening carrier (14) has a colour application (17).

13. The sealing tape according to any one of the preceding claims, **characterised in that** the colour application is applied to a nonwoven (82, 83, 163, 173) which is not inherently stable per se.

14. The sealing tape according to any one of the preceding claims, **characterised in that** a textile structure of the tape surface or the lamination has the colour application over the entire area.

15. The sealing tape according to any one of the preceding claims, **characterised in that** the colour application is applied to a surface of the fastening carrier.

16. The sealing tape according to any one of the preceding claims, **characterised in that** the colour application is applied to an additional and/or intermediate layer connected to the fastening carrier (14).

17. The sealing tape according to claim 16, **characterised in that** the colour application is applied to an additional layer and the sealing system component (6, 7) is placed on the additional layer (41, 52, 61).

18. The sealing tape according to claim 16 or 17, **characterised in that** the additional and/or intermediate layer comprises a film.

19. The sealing tape according to any one of the preceding claims, **characterised in that** the sealing system component has a hook region with a transparent base.

20. The sealing tape according to any one of the preceding claims, **characterised in that** the colour application is contoured and/or multicoloured.

21. Tape material (2) having a direction of longitudinal extension (9), which can be divided for individual uses (13) in the form of sealing tapes according to any one of the preceding claims by separating transversely to the direction of longitudinal extension (9) and possibly also substantially in the direction of longitudinal extension.

22. Nappy having a sealing tape according to any one of claims 1 to 20.

## Revendications

1. Bande de fermeture (13) comportant un support de fixation (14) comprenant une face avant textile ou revêtue de textile (3) et un composant (6) supporté par une face arrière (4) du support de fixation (14) dans une zone de fermeture d'un système de fermeture pour le raccordement dissociable à une surface, le support de fixation présentant un support élastique (81, 161, 171) qui est revêtu de non-tissé (82, 83, 163, 173), **caractérisée par** une application colorée (17) sur la face tournée vers le support élastique (81) du non-tissé (82, 83, 163, 173).

2. Bande de fermeture selon la revendication 1, **caractérisée en ce que** le support de fixation présente sur la face tournée vers le support élastique (81, 161, 171) du non-tissé (82, 83, 163, 173) une application colorée avec un recouvrement optique (138, 148, 158) pouvant être enlevé et agissant en direction de la face respective.

3. Bande de fermeture selon la revendication 2, **caractérisée en ce que** le recouvrement (138, 148, 158) peut être enlevé de manière irréversible.

4. Bande de fermeture selon la revendication 2, **caractérisée en ce que** le recouvrement (138, 148, 158) est fixé avec un point de rupture théorique (149), de sorte que le recouvrement peut être enlevé par un utilisateur sans éliminer l'application colorée ou au moins sans éliminer sensiblement les contours de l'application colorée.

5. Bande de fermeture selon une des revendications 2 à 4, **caractérisée en ce que** le recouvrement (138, 148, 158) présente une application colorée.

6. Bande de fermeture selon une des revendications précédentes, **caractérisée en ce que** le support élastique (81, 161, 171) est revêtu sur sa face avant et arrière de non-tissé (82, 83, 163, 173).

7. Bande de fermeture selon une des revendications précédentes, **caractérisée en ce que** l'application colorée est une impression.

8. Bande de fermeture selon une des revendications précédentes, **caractérisée en ce que** l'application colorée (17) est disposée à une extrémité utilisateur (15) de la bande de fermeture.

9. Bande de fermeture selon une des revendications précédentes, **caractérisée en ce que** l'application colorée (17) est disposée sur un ruban Fingerlift (22).

10. Bande de fermeture selon une des revendications précédentes, **caractérisée en ce que** l'application colorée (41, 52, 61) est disposée dans la zone de fermeture (45).

11. Bande de fermeture selon une des revendications précédentes, **caractérisée en ce que** l'application colorée (17) est disposée entre la zone de fermeture (45) et la zone de fixation (5, 42, 43).

12. Bande de fermeture selon une des revendications précédentes, **caractérisée en ce que** la face arrière (4) du support de fixation (14) présente une application colorée (17)

13. Bande de fermeture selon une des revendications précédentes, **caractérisée en ce que** l'application colorée (17) est apposée sur un non-tissé non stable en soi (82, 83, 163, 173).

14. Bande de fermeture selon une des revendications précédentes, **caractérisée en ce qu'**une structure textile de la surface de la bande ou du revêtement présente l'application colorée sur toute sa surface.

15. Bande de fermeture selon une des revendications précédentes, **caractérisée en ce que** l'application colorée est apposée sur une surface du support de fixation.

16. Bande de fermeture selon une des revendications précédentes, **caractérisée en ce que** l'application colorée (17) est apposée sur une couche supplémentaire et/ou intermédiaire raccordée au support de fixation (14).

17. Bande de fermeture selon la revendication 16, **caractérisée en ce que** l'application colorée (17) est apposée sur une couche supplémentaire et que le composant du système de fermeture (6, 7) est posé sur la couche supplémentaire (41, 52, 61).

18. Bande de fermeture selon la revendication 16 ou 17, **caractérisée en ce que** la couche supplémentaire et/ou intermédiaire présente un film.

19. Bande de fermeture selon une des revendications précédentes, **caractérisée en ce que** le composant du système de fermeture présente une zone de crochet dont la base est transparente.

20. Bande de fermeture selon une des revendications précédentes, **caractérisée en ce que** l'application colorée est cernée et/ou multicolore.

21. Bande de matériau (2) ayant un sens d'extension longitudinale (9) qui peut être divisée par sectionnement transversalement au sens d'extension longitudinale (9) et le cas échéant aussi sensiblement dans le sens d'extension longitudinale pour un usage individuel (13) sous forme de bandes de fermeture selon une des revendications précédentes.

22. Couche-culotte comportant une bande de fermeture selon une des revendications 1 à 20.
